(19) Europäisches Patentamt
European Patent Office
Office européen des brevets

(11) **EP 3 854 308 B1**

(12) **FASCICULE DE BREVET EUROPEEN**

(45) Date de publication et mention de la délivrance du brevet:
**03.05.2023 Bulletin 2023/18**

(21) Numéro de dépôt: **20153481.5**

(22) Date de dépôt: **23.01.2020**

(51) Classification Internationale des Brevets (IPC):
**A61B 7/00** (2006.01)     **A61B 5/024** (2006.01)

(52) Classification Coopérative des Brevets (CPC):
**A61B 7/00;** A61B 5/024

(54) **SYSTÈME DE PROCÉDÉ DE DÉTERMINATION D'UNE FRÉQUENCE CARDIAQUE PAR FILTRAGE PRÉDICTIF ET INTERCORRÉLATION DES SONS CAPTÉS SUR LA TRACHÉE D'UN INDIVIDU**

VERFAHRENSSYSTEM ZUR BESTIMMUNG EINER HERZFREQUENZ DURCH PRÄDIKTIVE FILTERUNG UND WECHSELBEZIEHUNG DER ERFASSTEN GERÄUSCHE AN DER LUFTRÖHRE EINES MENSCHEN

SYSTEM FOR METHOD FOR DETERMINING A CARDIAC FREQUENCY BY PREDICTIVE FILTERING AND INTERCORRELATION OF SOUNDS CAPTURED ON THE TRACHEA OF AN INDIVIDUAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**28.07.2021 Bulletin 2021/30**

(73) Titulaires:
- **Controle Instrumentation et Diagnostic Electroniques - CIDELEC**
  **49130 Sainte-Gemmes-sur-Loire (FR)**
- **Ecole Superieure Electronique de l'Ouest**
  **49100 Angers (FR)**

(72) Inventeurs:
- **FREYCENON, Nathalie**
  **49320 BRISSAC-LOIRE-AUBANCE (FR)**
- **LONGO, Roberto**
  **49100 ANGERS (FR)**

(74) Mandataire: **Vidon Brevets & Stratégie**
**16B, rue de Jouanet**
**BP 90333**
**35703 Rennes Cedex 7 (FR)**

(56) Documents cités:
**WO-A1-2005/096931     US-A1- 2006 287 606**
**US-A1- 2018 014 789     US-B1- 10 441 181**

**Description**

**1. DOMAINE DE L'INVENTION**

La présente invention concerne un procédé de détermination de la fréquence cardiaque d'un individu en utilisant les signaux sonores fournis par un microphone. L'invention concerne plus particulièrement le fait que les signaux sonores sont avant tout destinés à détecter des apnées du sommeil et que le procédé utilise les propriétés du filtrage prédictif et de l'intercorrélation.

**2. ARRIÈRE-PLAN TECHNOLOGIQUE**

Des individus peuvent être atteints d'une pathologie appelée « syndrome d'apnée du sommeil » qui consiste à arrêter la respiration pendant le sommeil. Ces absences de cycles respiratoires, d'une durée supérieure à 10 secondes, nuisent à la qualité du sommeil et provoquent une diminution du taux en oxygène du sang artériel. La diminution en oxygène détériore peu à peu les fonctions cérébrales et cardiaques, et la multiplication de micro-réveils empêche le cerveau d'entrer dans la phase de sommeil profond. Les individus atteints par cette pathologie ont un risque plus important d'accidents cardiovasculaires et de somnolence diurne responsable notamment d'accidents de la route ou du travail. Il importe donc de disposer d'un appareillage médical capable de détecter ce genre de pathologie chez des patients, afin de les informer de leur état et de les soigner.

L'analyse des troubles du sommeil repose sur l'exploration de la ventilation et des stades de sommeil. Pour détecter cette pathologie, un appareillage enregistre le comportement respiratoire du patient pendant son sommeil à l'aide de différents capteurs tels qu'un oxymètre de pouls, des canules nasales, des sangles thoraciques at abdominales, et un logiciel analyse les mesures afin d'identifier d'éventuels évènements pathologiques tels que des apnées et des hypopnées. La fréquence cardiaque peut être utile dans le dépistage ou le diagnostic du syndrome d'apnées du sommeil. En effet, celle-ci diminue à l'endormissement, fournissant ainsi une indication sur le sommeil du patient. Pendant les apnées du sommeil, la fréquence va encore diminuer, puis s'accélérer lors du micro-éveil qui suit généralement les apnées. En détectant de telles variations, et en les corrélant à une absence de son révélateur d'un arrêt de la ventilation, il est possible de confirmer le diagnostic.

La réduction du nombre de capteurs posés sur le patient durant la nuit est un enjeu important. La demande de brevet numéro FR1260334 déposée le 30 Octobre 2012 par la demanderesse divulgue un détecteur capable de déterminer les caractéristiques respiratoires d'un patient pendant son sommeil et de détecter la présence d'éventuelles apnées. Le détecteur comporte un microphone délivrant des signaux représentatifs du son entrant par la première ouverture et un capteur de pression délivrant des signaux représentatifs d'une pression statique. Un tel détecteur ne permet cependant pas une bonne précision pour calculer une fréquence cardiaque. En effet, le son capté peut être fortement bruité à cause notamment de ronflements, ou à cause des mouvements de l'individu au cours de son sommeil.

WO 2005/096931 porte sur une technique permettant d'estimer la fréquence respiratoire d'un patient. Le procédé consiste à dériver une pluralité de fréquences respiratoires à partir de sons respiratoires enregistrés sur un patient et à y appliquer un modèle mathématique de sélection fréquentielle prédéfinie.

**3. OBJECTIFS DE L'INVENTION**

Il existe donc un réel besoin d'un procédé d'analyse des sons captés par un détecteur placé sur un individu en train de dormir afin de déterminer la fréquence cardiaque et des informations relatives à sa ventilation ; les signaux acoustiques de la respiration sont ensuite corrélés avec les variations de fréquence cardiaque, pour confirmer ou infirmer la présence d'apnées du sommeil et/ou de hypopnées.

**4. PRESENTATION DE L'INVENTION**

Dans un mode de réalisation particulier de l'invention, il est proposé un procédé de détermination d'une fréquence cardiaque à l'aide d'un moyen de captation de son placé sur la trachée d'un individu, comprenant une étape préalable d'enregistrement des signaux sonores pendant une longue durée. Le procédé comporte en outre l'exécution ultérieure des étapes suivantes :

- filtrage des signaux numériques représentatifs des signaux sonores en utilisant un filtre adaptatif,
- application sur les signaux obtenus d'un filtre passe bas en vue de sélectionner des signaux compris dans une bande passante déterminée,
- sélection d'une première portion temporelle de signaux numériques filtrés d'une durée déterminée,
- intercorrélation avec une portion temporelle de signaux numériques filtrés qui suit dans le temps la première portion temporelle et qui est de même durée,

- recherche pour chaque portion de la position temporelle du maximum d'intercorrélation,
- calcul de l'intervalle de temps correspondant à cette position temporelle, et calcul de la fréquence cardiaque correspondante,
- répétition des étapes de sélection, d'intercorrélation, de recherche du maximum et de calcul de fréquence cardiaque sur toute la durée de l'enregistrement, pour obtenir une série temporelle de fréquences cardiaque Sf.

De cette manière, l'invention permet de mesurer au cours d'une longue période de mesure, par exemple une nuit entière, la fréquence cardiaque d'un patient et en corrélant les signaux acoustiques de la respiration avec des variations de fréquence cardiaque, il est possible d'en déduire la présence d'apnées du sommeil et/ou de hypopnées.

Selon un premier mode de réalisation, les étapes de sélection, d'intercorrélation et de calcul sont appliquées successivement sur les signaux numériques en décalant à chaque fois la fenêtre initiale d'une fraction de sa durée, aboutissant alors à plusieurs séries temporelles supplémentaires S2, S3... de fréquence cardiaque, et la fréquence cardiaque retenue pour construire la série temporelle finale Sf à un instant donné est choisie parmi les différentes séries temporelles. De cette manière, il est quand même possible de générer une intercorrélation même si les bruits B1 et B2 ne sont pas ou que partiellement présents dans les deux fenêtres.

Selon un autre mode de réalisation, le filtre adaptatif est un filtre prédictif.

Selon un autre mode de réalisation, la série temporelle finale Sf est construite en choisissant, pour chaque élément, la valeur dans la série temporelle pour laquelle la différence entre la valeur courante et la valeur médiane et la plus faible.

Selon un autre mode de réalisation, la durée déterminée des portions temporelles de signaux numériques filtrés est sensiblement une seconde.

Selon un autre mode de réalisation, le procédé comporte une étape de comparaison de chaque fréquence cardiaque avec la valeur moyenne calculée sur la période et une étape de remplacement de la valeur de fréquence par la valeur moyenne si la valeur de fréquence s'écarte au-delà d'un seuil de la valeur moyenne.

Selon un autre mode de réalisation, la détection et la correction d'une valeur aberrante consiste à construire une série temporelle de fréquence cardiaque temporaire St en filtrant la série temporelle de fréquence cardiaque finale Sf avec un filtre passe bas, et à remplacer les valeurs de la série temporelle Sf par celles de St lorsque l'écart entre ces deux valeurs est trop important.

Selon un autre mode de réalisation, si la fréquence cardiaque moyenne Fm est très éloignée de l'inverse de la durée de la portion temporelle choisie lors de l'étape de sélection d'une première portion temporelle, alors l'ensemble des opérations sont recommencées en adaptant la durée de portion temporelle à la fréquence moyenne Fm.

Selon un autre mode de réalisation, le moyen de captation de son est un microphone ou un capteur de pression.

## 5. DESCRIPTION DES FIGURES

D'autres caractéristiques et avantages de l'invention apparaîtront à la lecture de la description suivante, donnée à titre d'exemple indicatif et non limitatif, et des dessins annexés, dans lesquels :

- la figure 1 présente un individu dans une position de sommeil avec un détecteur de son relié à une unité de traitement ;
- la figure 2 présente un schéma d'un détecteur de son selon un exemple préféré de réalisation ;
- la figure 3 est un schéma illustrant la succession des bruits B1 à B4 émis par le coeur au cours d'un cycle cardiaque ;
- la figure 4 présente un ordinogramme des principales étapes du procédé de détermination d'une fréquence cardiaque, selon un exemple préféré de réalisation ;
- la figure 5 représente la structure d'un filtre linéaire à réponse impulsionnelle finie (FIR) utilisable pour la présente invention ;
- la figure 6 représente un schéma illustrant le principe d'un filtre prédictif utilisable pour la présente invention ;
- la figure 7 présente un organigramme d'une partie des étapes selon un exemple particulier de réalisation.

## 6. DESCRIPTION DETAILLEE D'UN MODE DE REALISATION

*6.1 Principe général*

Le principe général de l'invention repose sur un procédé de détermination d'une fréquence cardiaque à l'aide d'un moyen de captation de son placé sur la trachée d'un individu. Ce procédé comprend une étape préalable d'enregistrement des signaux sonores pendant une longue durée, une nuit par exemple. Un traitement est ensuite appliqué aux signaux enregistrés par les étapes suivantes : filtrage des signaux numériques représentatifs des signaux sonores en utilisant un filtre prédictif, application sur les signaux obtenus d'un filtre passe bas en vue de sélectionner des signaux compris dans une bande passante déterminée, sélection d'une première portion temporelle de signaux numériques filtrés d'une durée déterminée comportant deux signaux cardiaques appartenant à un même premier cycle cardiaque, détermination par intercorrélation avec une seconde portion temporelle de signaux numériques filtrés de même durée déterminée, et comportant également les deux mêmes signaux cardiaques appartenant à un second cycle cardiaque, calcul de la durée entre les instants séparant les mêmes signaux des deux portions temporelles, et détermination de la fréquence cardiaque en effectuant l'inverse de cette durée.

*6.2 Description d'un mode de réalisation*

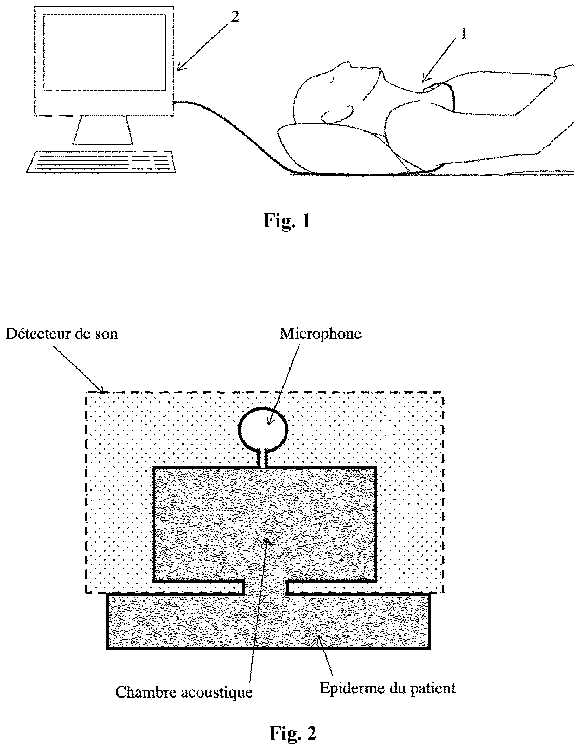

La **FIG.1** présente un individu dans une position de sommeil avec un détecteur de son relié à une unité de traitement. Le détecteur de son 1 est placé à la base du cou de l'individu au-dessus de sa trachée artère, il est éventuellement maintenu par un adhésif médical. Selon un mode préféré de réalisation, le détecteur 1 dispose d'un câble électrique relié à une unité de traitement 2 qui analyse les signaux sonores émis par l'individu et détermine ses caractéristiques respiratoires : inspiration, expiration, apnées, hypopnées, mouvement de la glotte, etc. Selon une variante de réalisation, le détecteur de son communique par une liaison sans fil à courte portée (de type Bluetooth par exemple).

D'un point de vue mécanique, l'épiderme de l'individu est similaire à une paroi élastique. Les flux d'air se déplaçant dans la gorge et la trachée artère génèrent des sons qui se propagent à travers les différents tissus de l'individu et sont parfaitement audibles à travers l'épiderme et à la surface de la peau.

La **FIG.2** présente un schéma d'un détecteur de son selon un exemple préféré de réalisation. La partie inférieure du détecteur est constituée d'une chambre acoustique débouchant sur un évent qui vient se poser sur l'épiderme du patient. Le bord de l'évent étant en contact avec l'épiderme, la chambre acoustique constitue une cavité fermée. De ce fait, les variations de pression dans la chambre acoustique dépendent des sons et de la pression provoqués par la respiration et les mouvements respiratoires. Un microphone est placé en communication acoustique avec la chambre pour détecter les ondes sonores. Pour mesurer une large gamme de fréquence acoustique, le microphone possède une bande passante assez large, s'étendant de 10 Hz à 10kHz. Le microphone détecte les sons à l'intérieur de la chambre acoustique, ces sons étant transmis par l'épiderme du patient, et les convertit en signaux électriques qui sont émis vers l'unité de traitement. Le microphone émet des signaux électriques représentatifs du son capté vers un Convertisseur Analogique Numérique (ou « CAN » en abrégé) qui est un circuit destiné à convertir des signaux analogiques en signaux numériques. Le convertisseur CAN échantillonne le signal analogique à une fréquence suffisamment haute pour capter les variations même fines du son et en déduire notamment les phases du cycle cardiaque en fonction des bruits émis par le coeur.

Le coeur est un muscle dont la fonction est de pomper le sang environ 100 000 fois par jour. Le coeur est composé de quatre parties, appelées des cavités. Les deux cavités supérieures s'appellent des oreillettes (ou atria), et les cavités inférieures des ventricules. Les quatre cavités communiquent entre elles au moyen de valvules, ou valves, qui s'ouvrent et se referment en fonction des variations de pression des différentes cavités cardiaques. Chaque oreillette est séparée du ventricule par une valvule : à droite la valvule tricuspide et à gauche, la valvule mitrale. La valve aortique sépare le ventricule gauche de l'aorte et la valve pulmonaire sépare le ventricule droit de l'aorte pulmonaire. Ces valves imposent comme des clapets anti-retours le sens de circulation du sang dans le coeur. Le côté gauche du coeur envoie le sang riche en oxygène, provenant de la veine pulmonaire vers la circulation systémique, pour le transporter vers tous les tissus de l'organisme hormis les alvéoles pulmonaires. Le côté droit du coeur pompe le sang pauvre en oxygène provenant des organes pour le transporter vers les alvéoles pulmonaires où il va se débarrasser du gaz carbonique et se recharger en oxygène.

Le fonctionnement du coeur est cyclique, le cycle cardiaque consiste en une succession de contractions et de relâchements du muscle, ainsi que d'ouvertures et fermetures de valves et de valvules. Un cycle cardiaque peut se diviser en deux périodes principales : une phase pendant laquelle se produit la contraction (systole) du ventricule et l'éjection du sang, qui alterne avec une phase de détente et de remplissage du ventricule (diastole).

L'activité cardiaque étant mécanique, elle produit des ondes sonores dont l'enregistrement, appelé phonocardiogramme est réalisé généralement sur le thorax coté antérieur par un stéthoscope électronique. Ces bruits mécaniques sont produits par le flux sanguin à travers les différentes cavités, les fermetures et les ouvertures des valves et valvules. Au cours d'un cycle cardiaque, quatre bruits sont émis par le coeur et sont nommés B1, B2, B3 et B4, en sachant que les bruits B1 et B2 ont des amplitudes plus élevées que celles de B3 et B4. C'est pourquoi la présente invention utilise la trace acoustique des bruits B1 et B2 pour déterminer la fréquence cardiaque.

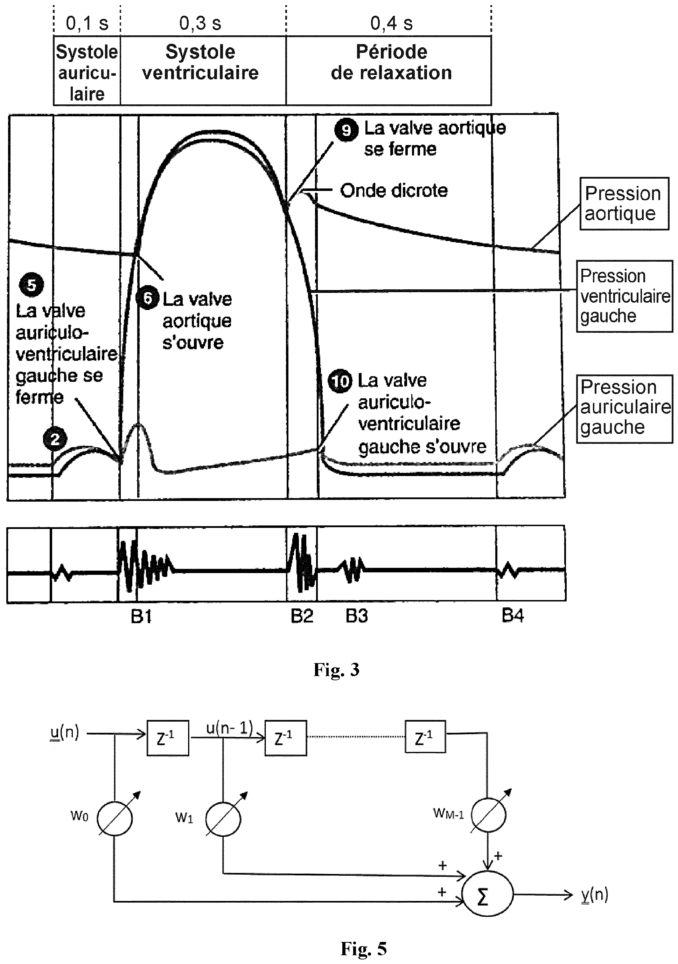

La **Fig. 3** illustre la succession des bruits B1 à B4 émis par le coeur au cours d'un cycle cardiaque. La **Fig. 3** comporte un bandeau supérieur présentant les trois phases principales : systole auriculaire, systole ventriculaire et période de relaxation, une partie centrale montrant les variations de pression au niveau de l'aorte, du ventricule gauche et de l'oreillette gauche, et un bandeau inférieur représentant un phonocardiogramme avec un repérage dans le temps des bruits B1 à B4.

Chacun des bruits possède une trace acoustique particulière et reconnaissable. La caractérisation de ces traces est décrite dans la thèse « Segmentation et classification des signaux non-stationnaires : application au traitement des sons cardiaque et à l'aide au diagnostic » produite par Ali Moukadem et soutenue le 16 Décembre 2011 à Mulhouse - France. On peut rappeler les caractéristiques essentielles de ces bruits. Le bruit B1 est un bruit résonant, plus intense et légèrement plus long que le deuxième bruit B2. Le bruit B1 possède deux composantes sonores : une valvulaire et une musculaire. La première composante est liée à la fermeture des valves auriculo-ventriculaires (tricuspide et mitrale), et la deuxième à la contraction brutale du muscle cardiaque, début de la systole ventriculaire. Le bruit B2 correspond à la fermeture des valves aortiques et pulmonaire au début de la diastole ventriculaire. Le bruit B3, possède une amplitude faible et est principalement audible chez l'enfant (jusqu'à 16 ans). Le bruit B3 correspond à la turbulence du sang pendant la phase de remplissage ventriculaire rapide. Le bruit B4 est émis lors de la phase de systole auriculaire qui consiste à remplir le ventricule par la contraction de l'oreillette. Pour un adulte sain, les bruits B3 et B4 sont quasiment inaudibles, leurs apparitions dans un enregistrement révèlent donc un signe pathologique. D'autres bruits anormaux tels que : le souffle cardiaque, des bruits diastoliques ou systoliques sont également des signes pathologiques.

Le bandeau supérieur de la **Fig. 3** montre que, pour un cycle cardiaque de 0,8 seconde correspondant à une fréquence cardiaque de 75 BPM (battements par minute), les différentes phases durent le temps suivant :

- la phase de systole auriculaire (entre B4 et B1) dure 0,1 seconde,
- la phase de systole ventriculaire (entre B1 et B2) dure 0,3 seconde,
- la période de relaxation (entre B2 et B4) dure 0,4 seconde.

L'invention permet notamment de détecter la présence de groupes de bruits B1 et B2 et de mesurer les durées qui séparent ces groupes, afin de déterminer des caractéristiques de fonctionnement cardiaque, et en particulier la fréquence. Cette détermination s'effectue par un module logiciel embarqué dans un ordinateur, tel que l'unité de traitement 2 qui reçoit les signaux numériques émis par Convertisseur CAN.

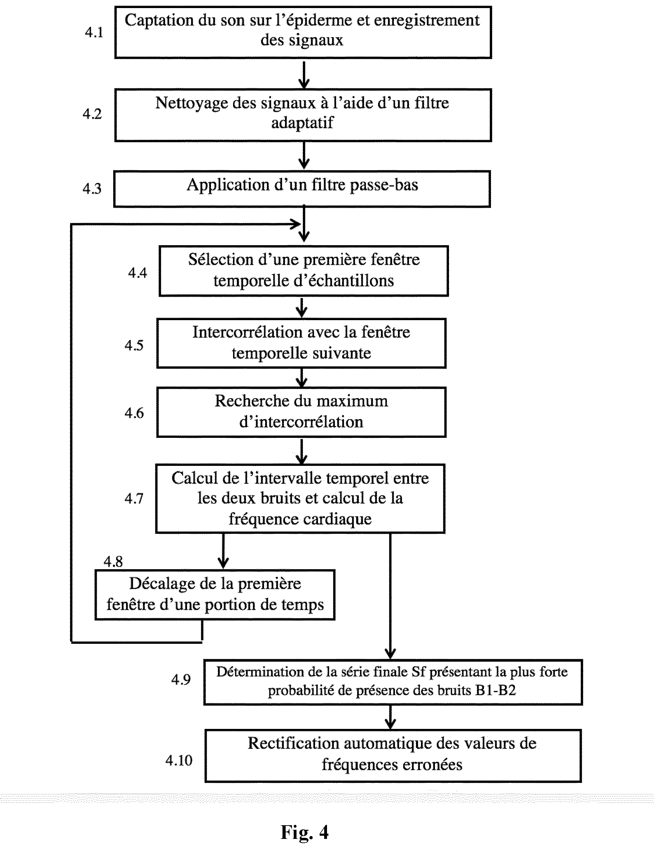

La **Fig. 4** présente un ordinogramme des principales étapes du procédé de détermination d'une fréquence cardiaque. Un patient est étendu sur un lit et un praticien de santé lui installe un détecteur de son au niveau de la trachée artère. Dans le cas d'une analyse liée à l'apnée, le recueil des signaux s'effectue pendant la nuit, lorsque le patient dort. A l'étape 4.1, le signal acoustique est capté par le microphone puis envoyé sous la forme de signaux électriques à un convertisseur CAN qui produit des signaux numériques. Ces signaux numériques sont enregistrés dans une mémoire informatique pour constituer les « données brutes ». Ces données peuvent être bruitées notamment à cause des ronflements du patient, ou à cause des mouvements de l'individu au cours de son sommeil. Les données brutes sont d'abord nettoyées en utilisant un filtre adaptatif de façon à diminuer l'importance du bruit (étape 4.2), ce filtre est avantageusement de type prédictif.

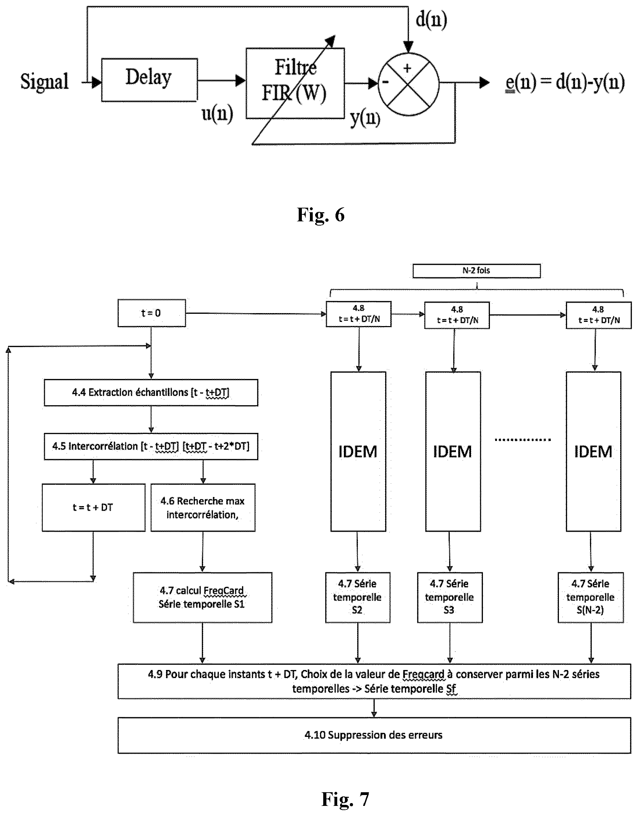

Le filtre prédictif présenté à la **Fig. 6.** est constitué des éléments suivants :
Un filtre FIR W de M coefficients, un delai Δ.

Les signaux sont réels, discrets et a l'instant n, on note les signaux suivants :

- $d(n)$ : la valeur du signal d'entrée (signal de référence),
- $u(n)$ : le signal d'entrée retardé $u(n) = d(n- \Delta)$,
- $y(n)$ : la sortie du filtre W,
- $e(n)$ : l'erreur estimée : $e(n) = d(n) - y(n)$.

$y(n)$ est la sortie du filtre prédictif.

Les M coefficients du filtre W sont définis par un processus itératif suivant l'algorithme Least-Mean-Square (LMS) proposé par Widrow et Hoff (références : Widrow, B., and Hoff, M.E., 1960, Adaptive switching circuits: IRE WESCON Conv. Rec., pt. 4, p 96-104. Widrow, B., and Stearns, S., 1985, Adaptive Signal Processing: Prentice-Hall, New York. ).

Les M coefficients du filtre W à l'instant n+1 sont alors déterminés par la relation :

$$\mathbf{W}(n+1) = \mathbf{W}(n) + 2\mu e(n)\mathbf{U}(n),$$

avec $W = [w_0 \ w_1 \cdots w_{M-1}]$
et

$$\mathbf{U}(n) = [u(n) \ u(n-1) \ \cdots \ u(n-M+1)]$$

Une façon alternative de calculer les coefficients optimaux du filtre W est de résoudre le problème classique

de Wiener :

$$\mathbf{W}_{opt} = \mathbf{R}^{-1}\mathbf{P}.$$

où R représente la matrice d'autocorrélation de l'entrée $\mathbf{U}(n)$ et P la matrice d'intercorrélation entre les entrées du filtre $\mathbf{U}(n)$ et le signal de référence d(n).

La solution de Wiener est détaillée ci-après.

La structure du filtre linéaire à réponse impulsionnelle finie (FIR) est représentée à la **Fig. 5.** L'objectif est de calculer les coefficients $\omega_k$ du filtre linéaire en recourant à l'erreur quadratique moyenne.

Cette erreur doit être minimale afin d'obtenir le meilleur filtre afin que la sortie du filtre se rapproche du mieux possible du signal désiré.

La sortie du filtre (FIR) y(n) s'écrit alors :

$$y(n) = \sum_{k=0}^{M-1} w_k u(n-k) = \mathbf{W}^T \mathbf{U}(n),$$

avec (le symbole T représente la transposée du vecteur)

$$\mathbf{W}^T = [w_0 \; w_1 \cdots w_{M-1}]$$

$$\mathbf{U}(n) = [u(n) \; u(n-1) \; \cdots \; u(n-M+1)]^T$$

L'erreur s'écrit alors :

$$e(n) = d(n) - y(n) = d(n) - \mathbf{W}^T \mathbf{U}(n).$$

L'erreur quadratique a donc pour expression :

$$e^2(n) = (d(n) - \mathbf{W}^T \mathbf{U}(n))^2 = d^2(n) + \mathbf{W}^T \mathbf{U}(n)\mathbf{U}^T(n)\mathbf{W} - 2d(n)\mathbf{W}^T \mathbf{U}(n)$$

En supposant le processus stochastique et stationnaire à l'ordre 2, l'erreur quadratique moyenne se calcule de la façon suivante :

$$E[e^2(n)] = \sigma_d^2 - 2\mathbf{P}^T \mathbf{W}(n) + \mathbf{W}^T(n)\mathbf{R}\mathbf{W}(n)$$

avec

$$\mathbf{P} \equiv \mathbf{P}_{dU} = E[d(n)\mathbf{U}(n)]$$

$$\mathbf{R} \equiv \mathbf{R}_{uu} = E[\mathbf{U}(n)\mathbf{U}^T(n)]$$

R représente ici la matrice d'autocorrélation de l'entrée U(n) et P la matrice d'intercorrélation entre les entrées du filtre et la sortie désirée. Il est ainsi possible de minimiser l'erreur quadratique moyenne en calculant les coefficients $\omega_k$ du filtre optimal. Pour cela, la solution de Wiener enseigne :

$$\frac{\partial E[e^2(n)]}{\partial w_k(n)} = 0$$

L'équation ci-dessus s'exprime de manière équivalente :

$$2\mathbf{RW}(n) - 2\mathbf{P}(n) = 0$$

En notant $W_{opt}$ la solution optimale, et en considérant la matrice R inversible, on peut conclure par l'expression suivante :

$$\mathbf{W}_{opt} = \mathbf{R}^{-1}\mathbf{P}.$$

A l'étape 4.3, les signaux nettoyés par le filtrage prédictif, par exemple par un algorithme prédictif de type LMS, passent à travers un filtre passe-bas selon une technique connue en soi, ce filtre passe-bas est par exemple du type Butterworth avec une fréquence de coupure à 35 Hz. En sortie du filtre passe-bas, les signaux numériques filtrés sont enregistrés et prêts à être traités.

Les mesures sont effectuées sur un patient qui est au repos, et qui dort de préférence. Dans ces conditions, la fréquence cardiaque de ce patient est comprise entre 40 BPM et 120 BPM. Le convertisseur CAN échantillonne le signal sonore à la fréquence de 4000 Hz (Fs), dans ces conditions un cycle cardiaque est couvert par un enregistrement de 2000 à 6000 échantillons de mesure. La trace acoustique d'un cycle cardiaque est selon la présente invention délimitée dans le temps entre les bruits B1-B2 d'un cycle cardiaque et le bruit B1-B2 du cycle cardiaque suivant. Pour évaluer ce temps, l'invention prévoit de mettre en oeuvre l'intercorrélation par fenêtre glissante qui est une méthode efficace pour déterminer l'écart de temps entre des motifs répétitifs dans un environnement susceptible d'être bruyant.

L'étape 4.4 consiste à extraire une première fenêtre d'un nombre déterminé d'échantillons des données obtenues par filtrage, cette première fenêtre possède donc une durée déterminée, typiquement une seconde. Le contenu de cette première fenêtre est corrélé à l'étape 4.5 avec la fenêtre suivante qui possède le même nombre déterminé de points, et donc de même durée. Si les bruits B1 et B2 sont présents dans les deux fenêtres successives, alors la position temporelle du maximum de l'intercorrélation informe du temps qui sépare un groupe de deux bruits B1 et B2 de la première fenêtre avec le groupe de deux bruits B1 et B2 de la deuxième fenêtre. La position temporelle du maximum d'intercorrélation est déterminée (étape 4.6). A l'étape 4.7, le procédé calcule l'intervalle de temps correspondant à cette position temporelle.

Puis, la fréquence cardiaque est alors calculée en effectuant l'inverse de cette durée. Multipliée par 60, cette valeur s'exprime en Battement Par Minute (BPM).

Cette opération est effectuée sur toute la durée de l'enregistrement, avec toutes les fenêtres temporelles successives. On obtient ainsi une série temporelle FreqCard(BMP). Lorsque les bruits B1 et B2 ne sont pas ou que partiellement présents dans les deux fenêtres, l'intercorrélation ne peut générer une bonne information. Pour pallier à cette difficulté, les opérations précédemment décrites sont répétées plusieurs fois en décalant la première fenêtre d'une fraction de la durée de celle-ci (étape 4.8). De cette manière, il est possible d'augmenter la probabilité de détecter une intercorrélation entre deux fenêtres contiguës présentant des bruits B1 et B2 successifs. A l'issue de ces opérations, on dispose de plusieurs séries temporelles S1, S2, S3, ..., qui ont chacune des valeurs bonnes et des valeurs fausses. Il découle de ce mode opératoire que ces séries temporelles sont échantillonnées à la fréquence 1/DT.

A l'étape 4.9, les différentes séries sont parcourues simultanément pour choisir parmi celles-ci quelle valeur conserver pour l'instant donné et pour générer une série temporelle unique Sf de fréquence cardiaque.

Dans un premier temps, des valeurs trop petites ou trop grandes peuvent être éliminées. Ensuite, différents moyens mettant en oeuvre des techniques de probabilité peuvent être utilisés, pour choisir à chaque instant dans quelle série temporelle S prendre la valeur de fréquence cardiaque.

Selon un mode préféré de réalisation, pour chaque série temporelle obtenues à l'issue des étapes 4.4 à 4.7 et en tenant compte du décalage (étape 4.8), le module calcule la valeur médiane d'ordre O (par exemple O = 17) autour de la valeur courante, ainsi que l'écart E entre la valeur courante et la valeur médiane. le module choisit alors la fréquence cardiaque de la série pour laquelle l'écart E est le plus faible.

La série temporelle unique Sf ainsi obtenue peut encore être entachée d'erreurs. Une dernière étape 4.10 peut alors être mise en oeuvre pour supprimer les erreurs. Différentes techniques peuvent être utilisées.

Selon un mode préféré de réalisation, le module calcule la valeur absolue de la différence entre la valeur précédente et la valeur suivante. Si ce nombre est plus petit qu'un certain seuil (par exemple 3 bpm), alors le module considère que la fréquence cardiaque est localement stable. La valeur courante ne doit pas trop s'éloigner de la valeur moyenne. Si la valeur courante s'en éloigne trop (par exemple 10 bpm), alors le module la remplace par la valeur moyenne.

Il est également possible de filtrer la série temporelle avec un filtre passe bas, par exemple un filtre passe bas du 4ieme ordre, avec une fréquence de coupure à 1/30 de la fréquence d'échantillonnage de la série temporelle S, c'est à dire : (1/DT) / 30. Ensuite, les valeurs de la série initiale qui s'écarteraient trop de la valeur filtrée sont remplacées par la valeur filtrée.

La **Fig. 7** présente un organigramme d'une partie des étapes selon un exemple particulier de réalisation. Les étapes de la **Fig. 4** y sont reproduites en précisant les instants d'exécution et la succession d'opération. On peut voir notamment les étapes composant la boucle aboutissant à générer chaque série temporelle S1, S2, S3, ... La recherche du maximum d'intercorrélation (étape 4.6) permet de déterminer l'intervalle de temps entre les positions temporelles des maxima d'intercorrélation de la première fenêtre et de la suivante, et d'en déduire la fréquence cardiaque en calculant l'inverse de cette durée (étape 4.7). Le traitement des données de chaque boucle s'effectue en décalant la première fenêtre d'une durée DT (étape 4.8). Pour chaque instant t + DT, la valeur de fréquence cardiaque à conserver est choisie parmi les séries temporelles ainsi générées pour construire la série temporelle finale Sf (étape 4.9). L'étape finale optionnelle 4.10 consiste à rectifier d'éventuelles erreurs en supprimant les fréquences erronées.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation et variantes doivent être considérés à titre d'illustration mais peuvent être modifiés dans le domaine défini par la portée des revendications jointes.

## Revendications

1. Procédé de détermination d'une fréquence cardiaque à l'aide d'un moyen de captation de son placé sur la trachée d'un individu, comprenant une étape préalable d'enregistrement (4.1) des signaux sonores pendant une longue durée, comprenant l'étape suivante :

   - filtrage (4.2) des signaux numériques représentatifs des signaux sonores en utilisant un filtre adaptatif, caractérisé en ce qu'il comporte ultérieurement les étapes suivantes :
   - application (4.3) sur les signaux obtenus par l'étape précédente (4.2) d'un filtre passe bas en vue de sélectionner des signaux compris dans une bande passante déterminée,
   - sélection (4.4) d'une première portion temporelle de signaux numériques filtrés d'une durée déterminée,
   - intercorrélation (4.5) avec une portion temporelle de signaux numériques filtrés qui suit dans le temps la première portion temporelle et qui est de même durée,
   - recherche (4.6) pour chaque portion de la position temporelle du maximum d'intercorrélation,
   - calcul de l'intervalle de temps correspondant à cette position temporelle, et calcul de la fréquence cardiaque correspondante (4.7),
   - répétition des étapes de sélection (4.4), d'intercorrélation (4.5), de recherche du maximum (4.6) et de calcul de fréquence cardiaque (4.7) sur toute la durée de l'enregistrement, pour obtenir une série temporelle de fréquences cardiaques Sf.

2. Procédé de détermination d'une fréquence cardiaque selon la revendication 1, **caractérisé en ce que** :

   - les étapes de sélection (4.4), d'intercorrélation (4.5) et de calcul (4.6) sont appliquées successivement sur les signaux numériques en décalant à chaque fois la fenêtre initiale d'une fraction de sa durée, aboutissant alors à plusieurs séries temporelles supplémentaires S2, S3... de fréquence cardiaque,
   - la fréquence cardiaque retenue pour construire la série temporelle finale Sf à un instant donné est choisie parmi les différentes séries temporelles (étape 4.9).

3. Procédé de détermination d'une fréquence cardiaque selon la revendication 1, **caractérisé en ce que** le filtre adaptatif est un filtre prédictif.

4. Procédé de détermination d'une fréquence cardiaque selon l'une quelconque des revendications précédentes, **caractérisé en ce que** la série temporelle finale Sf est construite en choisissant, pour chaque série temporelle, la valeur dans la série temporelle pour laquelle la différence entre la valeur courante et la valeur médiane et la plus faible.

5. Procédé de détermination d'une fréquence cardiaque selon l'une quelconque des revendications précédentes, **caractérisé en ce que** la durée déterminée des portions temporelles de signaux numériques filtrés est sensiblement une seconde.

6. Procédé de détermination d'une fréquence cardiaque selon l'une quelconque des revendications précédentes, **caractérisé en ce qu'**il comporte une étape de comparaison de chaque fréquence cardiaque avec la valeur moyenne calculée sur la période et une étape de remplacement de la valeur de fréquence par la valeur moyenne si la valeur de fréquence s'écarte au-delà d'un seuil de la valeur moyenne.

7. Procédé de détermination d'une fréquence cardiaque selon revendication 6, **caractérisée en ce que** la détection et la correction d'une valeur aberrante consiste à construire une série temporelle de fréquence cardiaque temporaire St en filtrant la série temporelle de fréquence cardiaque finale Sf avec un filtre passe bas, et à remplacer les valeurs de la série temporelle Sf par celles de St lorsque l'écart entre ces deux valeurs est trop important.

8. Procédé de détermination d'une fréquence cardiaque selon l'une quelconque des revendications précédentes, **caractérisé en ce que** si la fréquence cardiaque moyenne Fm est très éloignée de l'inverse de la durée de la portion temporelle choisie lors de l'étape de sélection (4.4), alors l'ensemble des opérations sont recommencées en adaptant la durée de portion temporelle à la fréquence moyenne Fm.

9. Procédé de détermination d'une fréquence cardiaque selon l'une quelconque des revendications précédentes, **caractérisé en ce que** ledit moyen de captation de son est un microphone ou un capteur de pression.

**Patentansprüche**

1. Verfahren zum Bestimmen einer Herzfrequenz mit Hilfe eines Schallerfassungsmittels, das auf der Luftröhre einer Person angeordnet wird, umfassend eine Vorabschritt des Aufzeichnens (4.1) der Schallsignale über einen langen Zeitraum, umfassend den folgenden Schritt:

   - Filtern (4.2) der digitalen Signale, die für die Schallsignale repräsentativ sind, unter Verwendung eines adaptiven Filters,

   **dadurch gekennzeichnet, dass** es ferner die folgenden Schritte aufweist:

   - Anwenden (4.3) eines Tiefpassfilters auf die Signale, die durch den vorherigen Schritt (4.2) erhalten werden, um Signale auszuwählen, die in einer bestimmten Bandbreite enthalten sind,
   - Auswählen (4.4) eines ersten zeitlichen Abschnitts gefilterter digitaler Signale einer bestimmten Dauer,
   - Kreuzkorrelieren (4.5) mit einem zeitlichen Abschnitt gefilterter digitaler Signale, der zeitlich auf den ersten zeitlichen Abschnitt folgt und von gleicher Dauer ist,
   - Suchen (4.6) des Maximums der Kreuzkorrelation für jeden zeitlichen Abschnitt,
   - Berechnen des Zeitintervalls, das dieser zeitlichen Position entspricht, und Berechnen der entsprechenden Herzfrequenz (4.7),
   - Wiederholen der Schritte des Auswählens (4.4), des Kreuzkorrelierens (4.5), des Suchens des Maximums (4.6) und des Berechnens der Herzfrequenz (4.7) für die gesamte Dauer des Aufzeichnens, um eine Zeitreihe von Herzfrequenzen Sf zu erhalten.

2. Verfahren zum Bestimmen einer Herzfrequenz nach Anspruch 1, **dadurch gekennzeichnet, dass**:

   - die Schritte des Auswählens (4.4), des Kreuzkorrelierens (4.5) und des Berechnens (4.6) nacheinander auf die digitalen Signale angewendet werden, wobei jedes Mal das Ausgangsfenster um einen Bruchteil seiner Dauer verschoben wird, wodurch sich dann mehrere zusätzliche Zeitreihen S2, S3 ... der Herzfrequenz ergeben,
   - die Herzfrequenz, die gewählt wird, um die endgültige Zeitreihe Sf zu einem gegebenen Zeitpunkt zu konstruieren, aus den verschiedenen Zeitreihen ausgewählt wird (Schritt 4.9).

3. Verfahren zum Bestimmen einer Herzfrequenz nach Anspruch 1, **dadurch gekennzeichnet, dass** der adaptive Filter ein prädiktiver Filter ist.

**4.** Verfahren zum Bestimmen einer Herzfrequenz nach einem der vorhergehenden Ansprüche, **dadurch gekennzeichnet, dass** die endgültige Zeitreihe Sf konstruiert wird, indem für jede Zeitreihe der Wert in der Zeitreihe ausgewählt wird, für den die Differenz zwischen dem aktuellen Wert und dem Medianwert die geringste ist.

**5.** Verfahren zum Bestimmen einer Herzfrequenz nach einem der vorhergehenden Ansprüche, **dadurch gekennzeichnet, dass** die bestimmte Dauer der zeitlichen Abschnitte gefilterter digitaler Signale im Wesentlichen eine Sekunde beträgt.

**6.** Verfahren zum Bestimmen einer Herzfrequenz nach einem der vorhergehenden Ansprüche, **dadurch gekennzeichnet, dass** es einen Schritt des Vergleichens jeder Herzfrequenz mit dem Mittelwert, der über den Zeitraum berechnet wird, und einen Schritt des Ersetzens des Frequenzwerts durch den Mittelwert aufweist, wenn der Frequenzwert über einen Schwellenwert des Mittelwerts hinaus abweicht.

**7.** Verfahren zum Bestimmen einer Herzfrequenz nach Anspruch 6, **dadurch gekennzeichnet, dass** das Erkennen und Korrigieren eines Ausreißers darin besteht, eine temporäre Herzfrequenzzeitreihe St zu konstruieren, indem die endgültige Herzfrequenzzeitreihe Sf mit einem Tiefpassfilter gefiltert wird und die Werte der Zeitreihe Sf durch die von St ersetzt werden, wenn der Unterschied zwischen diesen zwei Werten zu groß ist.

**8.** Verfahren zum Bestimmen einer Herzfrequenz nach einem der vorhergehenden Ansprüche, **dadurch gekennzeichnet, dass**, falls die durchschnittliche Herzfrequenz Fm sehr weit vom Kehrwert der Dauer des zeitlichen Abschnitts, der während des Schritts des Auswählens (4.4) ausgewählt wird, entfernt ist, alle Operationen dann wiederholt werden, indem die Dauer des zeitlichen Abschnitts an die durchschnittliche Frequenz Fm angepasst wird.

**9.** Verfahren zum Bestimmen einer Herzfrequenz nach einem der vorhergehenden Ansprüche, **dadurch gekennzeichnet, dass** das Schallerfassungsmittel ein Mikrofon oder ein Drucksensor ist.

## Claims

**1.** A method for determining a heart rate using a sound sensing means placed on an individual's trachea, comprising a prior step of recording (4.1) sound signals for a long duration, comprising the following step:

- filtering (4.2) digital signals representative of the sound signals by using an adaptive filter,

**characterised in that** it subsequently includes the following steps:

- applying (4.3) to the signals obtained by the preceding step (4.2) a low pass filter in order to select signals included in a determined pass-band,
- selecting (4.4) a first time portion of filtered digital signals having a determined duration,
- intercorrelating (4.5), with a time portion, filtered digital signals which follows in time the first time portion and which is of the same duration,
- searching (4.6) for the maximum intercorrelation for each portion of the time position,
- calculating the time interval corresponding to this time position, and calculating the corresponding heart rate (4.7),
- repeating the selection (4.4), intercorrelation (4.5), maximum search (4.6) and heart rate calculation (4.7) steps over the entire duration of recording, to obtain a time series of heart rates Sf.

**2.** The method for determining a heart rate according to claim 1, **characterised in that**:

- the selection (4.4), intercorrelation (4.5) and calculation (4.6) steps are successively applied to the digital signals by offsetting each time the initial window by a fraction of its duration, then resulting in several additional time series S2, S3... of heart rate,
- the heart rate retained to construct the final time series Sf at a given time instant is chosen among the different time series (step 4.9).

**3.** The method for determining a heart rate according to claim 1, **characterised in that** the adaptive filter is a predictive filter.

4. The method for determining a heart rate according to any of the preceding claims, **characterised in that** the final time series Sf is constructed by choosing, for each time series, the value in the time series for which the difference between the current value and the median value is the smallest.

5. The method for determining a heart rate according to any of the preceding claims, **characterised in that** the determined duration of the time portions of filtered digital signals is substantially one second.

6. The method for determining a heart rate according to any of the preceding claims, **characterised in that** it includes a step of comparing each rate with the mean value calculated over the period and a step of replacing the rate value with the mean value if the rate value deviates beyond a threshold from the mean value.

7. The method for determining a heart rate according to claim 6, **characterised in that** the detection and the correction of an outlier consists in constructing a time series of temporary heart rate St by filtering the time series of final heart rate Sf with a low pass filter, and in replacing the values of the time series Sf by those of St when the deviation between these two values is too large.

8. The method for determining a heart rate according to any of the preceding claims, **characterised in that** if the mean heart rate Fm is very far from the reciprocal of the duration of the time portion chosen during the selection step (4.4), then all the operations are started again by adapting the duration of the time portion to the mean rate Fm.

9. The method for determining a heart rate according to any of the preceding claims, **characterised in that** said sound sensing means is a microphone or a pressure sensor.

**Fig. 1**

Détecteur de son

Microphone

Chambre acoustique

Epiderme du patient

**Fig. 2**

| 0,1 s | 0,3 s | 0,4 s |
|---|---|---|
| Systole auricu- laire | Systole ventriculaire | Période de relaxation |

**❾** La valve aortique se ferme

— Onde dicrote

Pression aortique

Pression ventriculaire gauche

**❺** La valve auriculo- ventriculaire gauche se ferme

**❻** La valve aortique s'ouvre

**❷**

**❿** La valve auriculo- ventriculaire gauche s'ouvre

Pression auriculaire gauche

B1          B2  B3          B4

**Fig. 3**

$\underline{u}(n)$ → $Z^{-1}$ — $u(n-1)$ → $Z^{-1}$ ....... $Z^{-1}$

$W_0$     $W_1$     $W_{M-1}$

$+$
$+$
$+$
$\Sigma$ → $\underline{y}(n)$

**Fig. 5**

```
4.1  ┌─────────────────────────────────────────┐
     │ Captation du son sur l'épiderme et      │
     │ enregistrement des signaux              │
     └─────────────────────────────────────────┘
                        │
                        ▼
4.2  ┌─────────────────────────────────────────┐
     │ Nettoyage des signaux à l'aide d'un     │
     │ filtre adaptatif                        │
     └─────────────────────────────────────────┘
                        │
                        ▼
4.3  ┌─────────────────────────────────────────┐
     │ Application d'un filtre passe-bas       │
     └─────────────────────────────────────────┘
                        │
                        ▼
4.4  ┌─────────────────────────────────────────┐
     │ Sélection d'une première fenêtre        │
     │ temporelle d'échantillons               │
     └─────────────────────────────────────────┘
                        │
                        ▼
4.5  ┌─────────────────────────────────────────┐
     │ Intercorrélation avec la fenêtre        │
     │ temporelle suivante                     │
     └─────────────────────────────────────────┘
                        │
                        ▼
4.6  ┌─────────────────────────────────────────┐
     │ Recherche du maximum                    │
     │ d'intercorrélation                      │
     └─────────────────────────────────────────┘
                        │
                        ▼
4.7  ┌─────────────────────────────────────────┐
     │ Calcul de l'intervalle temporel entre   │
     │ les deux bruits et calcul de la         │
     │ fréquence cardiaque                     │
     └─────────────────────────────────────────┘
                        │
                        ▼
4.8  ┌─────────────────────────────────────────┐
     │ Décalage de la première                 │
     │ fenêtre d'une portion de temps          │
     └─────────────────────────────────────────┘

4.9  ┌─────────────────────────────────────────┐
     │ Détermination de la série finale Sf     │
     │ présentant la plus forte probabilité    │
     │ de présence des bruits B1-B2            │
     └─────────────────────────────────────────┘
                        │
                        ▼
4.10 ┌─────────────────────────────────────────┐
     │ Rectification automatique des valeurs   │
     │ de fréquences erronées                  │
     └─────────────────────────────────────────┘
```

**Fig. 4**

**Fig. 6**

**Fig. 7**

## RÉFÉRENCES CITÉES DANS LA DESCRIPTION

**Documents brevets cités dans la description**

- FR 1260334

- WO 2005096931 A

**Littérature non-brevet citée dans la description**

- **ALI MOUKADEM.** *Segmentation et classification des signaux non-stationnaires : application au traitement des sons cardiaque et à l'aide au diagnostic,* 16 Décembre 2011

- **WIDROW, B. ; HOFF, M.E.** Adaptive switching circuits: IRE WESCON Conv. Rec. 1960, vol. 4, 96-104
- **WIDROW, B. ; STEARNS, S.** Adaptive Signal Processing. Prentice-Hall, 1985